# EUROPÄISCHE PATENTSCHRIFT

(11) **EP 1 536 770 B1**
(45) Veröffentlichungstag und Bekanntmachung des Hinweises auf die Patenterteilung: **29.07.2015**
(21) Anmeldenummer: 03753411.2
(22) Anmeldetag: 12.09.2003
(51) Int. Cl.: A61K 9/127, A61K 49/00, A61K 31/00

(54) **THERMOLABILES LIPOSOM MIT GEREGELTER FREIGABETEMPERATUR**
THERMOLABILE LIPOSOME WITH A CONTROLLED RELEASE TEMPERATURE
LIPOSOME THERMOLABILE A TEMPERATURE DE LIBERATION REGULEE

(30) Priorität: 12.09.2002 DE 10242367
(43) Veröffentlichungstag der Anmeldung: 08.06.2005
(73) Patentinhaber: Max-Planck-Gesellschaft zur Förderung der Wissenschaften e.V., 80539 München (DE)
(72) Erfinder: EIBL, Hansjörg, 37120 Bovenden (DE); LINDNER, Lars, H., 81377 München (DE)
(74) Vertreter: Weickmann & Weickmann
(86) Internationale Anmeldenummer: PCT/EP2003/010163
(87) Internationale Veröffentlichungsnummer: WO 2004/026282

(56) Entgegenhaltungen:
- WO-A-02/064116
- WO-A-03/026617
- DE-A- 19 622 224
- MARUYAMA K ET AL: "PHOSPHATIDYL POLYGLYCEROLS PROLONG LIPOSOME CIRCULATION IN VIVO" INTERNATIONAL JOURNAL OF PHARMACOGNOSY, SWETS & ZEITLINGER, LISSE, NL, Bd. 111, 1994, Seiten 103-107, XP000672277 ISSN: 0925-1618
- WHITEMAN, K.; MACKIE, S.; LEVCHENKO, T.; HOFFMANN, P.; TORCHILIN, V.: 'Long-circulating liposomes with phosphatidyl-(oligo)-glycerols' PROCEEDINGS - 28TH INTERNATIONAL SYMPOSIUM ON CONTROLLED RELEASE OF BIOACTIVE MATERIALS, CONTROLLED RELEASE SOCIETY Nr. 28, 23 Juni 2001, Seiten 466 - 467, XP009025848
- LEHNERT, RENE; EIBL, HANS-JOERG; MUELLER, KLAUS: 'FT-IR and NMR Studies on the Conformational and Structural Properties of 1,2-Dipalmitoyl-sn-glycero-3- phosphatidyloligoglycerols' JOURNAL OF PHYSICAL CHEMISTRY B Bd. 107, Nr. 1, 2003, Seiten 75 - 85, XP009025885

## Beschreibung

Die Erfindung betrifft ein thermolabiles Liposom mit geregelter Freigabetemperatur für den Liposomeninhalt, insbesondere ein bei 37 °C in Serum stabiles Liposom mit einer geregelten Freigabetemperatur zwischen 40 und 80 °C.

Liposomen sind künstlich gebildete Vesikel aus Lipiddoppelschichten, welche ein wässriges Kompartiment einschließen (Bangham et al., 1965). Ursprünglich noch als Modellsystem für eine Zellmembran genutzt, wurden Liposomen in jüngster Zeit vor allem für den Arzneistofftransport weiterentwickelt. Liposomen können dabei die Verträglichkeit von Wirkstoffen steigern (Senkung der aktiven Toxizität von Amphothericin B durch liposomale Formulierung (AmBisome®) um den Faktor 75 (Proffitt et al., 1991)). Sie eröffnen aber auch die Möglichkeit, Arzneistoffe gezielt in erkranktes Gewebe zu transportieren (Forssen et al., 1992). Nach intravenöser Applikation werden Liposomen hauptsächlich in Zellen des retikuloendothelialen Systems (RES) der Leber und Milz aufgenommen (Gregoriadis und Nerunhun, 1974). Um Liposomen als Arzneistoffträger für Zellen außerhalb des RES nutzen zu können, versuchte man die Zirkulationszeit der Liposomen im Blut zu erhöhen. Vor allem in Tumoren, die häufig sehr gut vaskularisiert sind (Jain, 1996) und deren Gefäße durch geweitete interendotheliale Verbindungen, eine große Anzahl von Fenestrierungen sowie diskontinuierliche Basalmembranen (Murray and Carmichael, 1995) besonders durchlässig sind, würde sich die Aufnahmewahrscheinlichkeit von Liposomen dadurch massiv erhöhen.

WO 02/064116 A2 beschreibt thermolabile Liposomen mit geregelter Freigabetemperatur für den Liposomeninhalt, welche im Wesentlichen aus mindestens einem Phosphatidylcholin mit einer Hauptumwandlungstemperatur, die im Bereich von 0 bis 80 °C liegt, und 2 bis 15 Gew.-% Phosphatidyloligoglycerin gebildet sind.

DE 196 22 224 A1 beschreibt Liposomen mit verlängerter Halbwertszeit im Blut, welche Phosphatidyloligoglycerine enthalten.

K. Maruyama et al., International Journal of Pharmaceutics 111 (194) 103-107 beschreibt Liposomen, umfassend Distearoylphosphatidylcholin, Cholesterin und verschiedene Mengen an Dipalmitoylphosphatidyloligoglycerin.

K. Whiteman et al., Proceed. Int'l. Symp. Control. Rel. Bioact. Mater., 28 (2001), 166 beschreibt Liposomen, umfassend Phosphatidylcholin und Cholesterin sowie Distearoylphosphatidyl-diglycerin oder Distearoylphosphatidyl-triglycerin.

Ein erstes Problem bei der Verwendung von Liposomen zum Transport von Wirkstoffen oder Markierungsstoffen in Körperflüssigkeiten liegt in der Erhöhung der Zirkulationszeit im Serum. Man hat zwar bereits gefunden, dass durch kovalente Bindung von Methoxypolyethylenglykolen an die Liposomenmembran die frühzeitige Erkennung der Liposomen durch das RES verhindert wird und damit die Zirkulationszeit der Liposomen verbessert werden kann. Neben einer Verbesserung der Zirkulationszeit besteht jedoch auch ein großes Interesse an einer Möglichkeit, durch Temperatureinwirkung eine gezielte Freigabe der Liposomeninhaltsstoffe bei bestimmter Temperatur zu erreichen.

Der Erfindung liegt daher die Aufgabe zugrunde, ein Liposom bereitzustellen, welches eine wesentlich verbesserte Halbwertszeit im Serum aufweist, verglichen mit der üblichen Halbwertszeit bekannter Liposomen in der Größenordnung um 4 Stunden, und welches so beschaffen ist, dass der Inhalt der Liposomen bei einer bestimmten Temperatur rasch freigesetzt wird.

Gelöst wird diese Aufgabe gemäß der vorliegenden Erfindung durch ein Liposom mit geregelter Freigabetemperatur für den Liposomeninhalt, welches dadurch gekennzeichnet ist, dass es im Wesentlichen aus mindestens einem Phosphatidylcholin mit einer Hauptumwandlungstemperatur im Bereich von 0 bis 80 °C und mehr als 15 bis 70 Gew.-% Phosphatidyloligoglycerin gebildet ist und dass es kein Cholesterin enthält. Gemäß einem älteren Vorschlag war es lediglich möglich, Liposomen mit einem maximalen Phosphatidyloligoglycerin-Gehlt von 15 Gew.-% zu erhalten. Nunmehr wurde jedoch überrschenderweise gefunden, dass es möglich ist, den Phosphatidyloligoglyceringehalt bis zu 70 % zu erhöhen, sodass der Bereich der erzielbaren Freigabetemperaturen der Liposomen noch mehr erweitert wird, aber vor allem die Halbwertszeiten nochmals verbessert werden.

Gemäß einer bevorzugten Ausführungsform enthalten die erfindungsgemäßen Liposomen zusätzlich geringere Mengen an Alkylphosphocholinen, vorzugsweise 10 bis 15 Gew.-%. Geeignete Substanzen sind z.B. Hexadecylphosphocholin, Oleylphosphocholin sowie Etherlysolecithine. Bei den Etherlysolecithinen kann die Hydroxylgruppe in Position 2 des Glycerins methyliert oder frei vorliegen. Bei dieser Ausführungsform gelingt es, die Freisetzung der im Liposom eingeschlossenen Substanzen von etwa 70 % ohne den Gehalt an Alkylphosphocholin auf praktisch 100 % zu erhöhen, was auf eine Beschleunigung der Liposomenöffnung zurückzuführen ist. Des Weiteren weisen die Alkylphosphocholine einen antitumoralen Effekt durch temperaturabhängige Freisetzung aus den Liposomen auf.

Erfindungsgemäß aufgebaute Liposomen weisen wesentlich verbesserte Halbwertszeiten von bis zu mehr als 25 Stunden im Serum auf und können durch geeignete Wahl der Komponenten und Mengen der Komponenten in Abhängigkeit von deren Hauptumwandlungstemperatur den (oder die) Inhaltsstoff(e) bei einer vorbestimmten Temperatur rasch und vollständig freigeben.

Bevorzugt ist das erfindungsgemäße Liposom aus etwa 20 bis 75 Gew.-% Dipalmitoyllecithin (1,2-Dipalmitoylglycero-3-phosphocholin), etwa 10 bis 25 Gew.-% Distearoyllecithin (1,2-Distearoylglycero-3-phosphocholin) und mehr als 15 bis etwa 50 Gew.-% Dipalmitoylphosphoglyceroglycerin zusammengesetzt. Eine solche bevorzugte Zusammensetzung ist bei 37 °C im Serum stabil, gibt jedoch den Inhalt bei Überschreiten einer Temperatur von 40 °C rasch frei.

Eine weitere bevorzugte Zusammensetzung mit verbesserter Freigabe der im Liposom eingeschlossenen Substanzen besteht aus etwa 15 bis 70 Gew.-% Dipalmitoyllecithin, etwa 10 bis 25 Gew.-% Distearoyllecithin und mehr als 15 bis etwa 45 Gew.-% Dipalmitoylphosphoglyceroglycerin.

Die vorstehend genannte bevorzugte Zusammensetzung des erfindungsgemäßen Liposoms lässt sich für andere Temperaturbereiche maßschneidern durch Wahl von Komponenten mit der jeweils geeigneten Hauptumwandlungstemperatur. In Tabelle 1 sind die Hauptumwandlungstemperaturen (T_{M}) von Phosphatidylcholinen angegeben, deren Hauptumwandlungstemperaturen im Bereich von 0 bis 80 °C liegen. Die Hauptumwandlungstemperaturen sind wie aus der Tabelle erkennbar, abhängig von der Kettenlänge und der Verteilung über die Positionen 1 und 2 von Glycero-3-phosphocholin oder über die Positionen 1 und 3 von Glycero-2-phosphocholin.

**Tabelle 1**

| T_{M} | Phosphatidylcholin |
|---|---|
| 5 °C | 1-Palmitoyl-2-oleoyl- |
| 7 °C | 1-Stearoyl-2-oleoyl- |
| 11 °C | 1-Palmitoyl-2-lauroyl- |
| 14 °C | 1-Behenoyl-2-oleoyl- |
| 17 °C | 1-Stearoyl-2-lauroyl- |
| 19 °C | 1,3-Dimyristoyl- |
| 23 °C | 1,2-Dimyristoyl- |
| 27 °C | 1-Palmitoyl-2-myristoyl- |
| 33 °C | 1-Stearoyl-2-myristoyl- |
| 37 °C | 1-Myristoyl-2-palmitoyl- |
| 39 °C | 1,3-Dipalmitoyl- |
| 41 °C | 1,2-Dipalmitoyl- |
| 42 °C | 1-Myristoyl-2-stearoyl- |
| 46 °C | 1-Stearoyl-3-myristoyl- |
| 48 °C | 1-Stearoyl-2-palmitoyl- |
| 52 °C | 1-Palmitoyl-2-stearoyl- |
| 53 °C | 1,3-Distearoyl- |
| 56 °C | 1,2-Distearoyl- |
| 66 °C | 1,2-Diarachinoyl- |
| 75 °C | 1,2-Dibehenoyl- |
| 80 °C | 1,2-Dilignoceroyl- |

Die in der Tabelle 1 aufgeführten Werte zeigen, dass durch Verwendung von Fettsäuren mit ungerader Kettenlänge und geeigneter Verteilung über das Glyceringrundgerüst praktisch jede gewünschte Temperatur im angegebenen Bereich von 0 bis 80 °C eingestellt werden kann.

Der Gehalt an Phosphatidyloligoglycerinen im erfindungsgemäßen Liposom ist essenziell für die erforderliche lange Zirkulationszeit im Serum. Phosphatidyloligoglycerine und ihre Herstellung sind bekannt aus der DE 196 22 224. Bevorzugt wird Dipalmitoylphosphoglyceroglycerin (DPPG2) verwendet.

Die erfindungsgemäßen thermolabilen Liposomen eignen sich hervorragend für die Anwendung auf verschiedenen Gebieten, insbesondere aber im Rahmen der regionalen Tiefenhyperthermie. Die regionale Tiefenhyperthermie, die in Kombination mit systemischer Chemotherapie an spezialisierten klinischen Zentren angewendet wird, bietet sich als ideale Technik für den tumorspezifischen liposomalen Transport und die anschließende Freisetzung eines Arzneistoffs aus der liposomalen Hülle an. So fördert die Hyperthermie zum einen die Extravasation von Liposomen aus Tumorkapillaren in das Interstitium (Gaber et al., 1996). Zum anderen kann durch die Erwärmung eine Freisetzung des Arzneistoffs aus speziellen thermosensitiven Liposomen induziert werden (Magin und Niesman, 1984). Zusätzlich gibt es zahlreiche Hinweise für einen gesteigerten zytotoxischen Effekt von Zytostatika (Hahn et al., 1975) sowie einer Immunmodulation (Aktivierung von NK-Zellen; Multhoff et al., 1999) durch regionale Tiefenhyperthermie.

Die Thermolabilität der erfindungsgemäßen Liposomen wird durch die Phasenumwandlung der Phospholipide innerhalb der Liposomenmembran bedingt. Wird die Phasenumwandlungstemperatur durchlaufen, so kommt es zu einer kurzzeitigen Membraninstabilität und anschließenden Freisetzung des liposomalen Inhalts.

Bei der oben erwähnten regionalen Hyperthermie wird der Tumor regional spezifisch überwärmt, sodass die Temperatur über der Grenztemperatur zur Freisetzung des Liposomeninhalts ansteigt. Als Liposomeninhalt kommen hierbei insbesondere in der Onkologie anwendbare Wirkstoffe, wie z.B. Zytostatika, in Betracht. Jedoch können auch Kontrastmittel, beispielsweise Gadolinium, z.B. Magnevist^{®}, Multihance^{®} oder Omniscan^{®}, Carboxyfluorescein, jodhaltige Kontrastmittel, die sich von Pyridinen oder aromatischen Carbonsäuren ableiten, o.dgl. allein oder zusammen mit einem Wirkstoff zur Freisetzung gebracht werden. Dietemperaturabhängige Freisetzung von Gadolinium aus den Liposomen lässt sich durch eine veränderte T1-Zeit mit Hilfe eines MRT darstellen (0,2 bzw. 1,5 Teslar). Durch Verwendung von Kontrastmitteln, wie Gadolinium, wird eine nicht invasive Thermometrie möglich gemacht, bei der die erreichte Temperatur durch MRC bestimmt werden kann, die das freigesetzte Gadolinium misst. Bei dieser Anwendung der erfindungsgemäßen Liposomen wird zweckmäßig ein Hyperthermiegerät mit einem MRC-Gerät gekoppelt angewendet. Eine Verwendung von Liposomen mit jodhaltigem Kontrastmittel für die Darstellung in der Computertomographie (beispielsweise für die Thermoablation von Lebermetastasen) ist auch denkbar.

Eine weitere Anwendungsart für die erfindungsgemäßen Liposomen findet sich in der Augenheilkunde. Bei Einkapselung einer fluoreszierenden Markierungssubstanz lässt sich z.B. bei einer Laserbehandlung durch Freisetzung des fluoreszierenden Wirkstoffes, wie z.B. Carboxyfluorescein, nachweisen, wo die angestrebte Überwärmung tatsächlich aufgetreten ist.

Analog zu der am Auge erläuterten Einsatzmöglichkeit können daher erfindungsgemäße Liposomen generell dazu verwendet werden, erreichte Temperaturen nachträglich bestimmbar zu machen, z.B. wenn bestimmte Erhitzungstemperaturen o.dgl. festgestellt werden sollen.

Die erfindungsgemäßen Liposomen bestehen im Wesentlichen aus den oben angegebenen Substanzen, die bevorzugt in reiner Form vorliegen. Verunreinigungen sollten möglichst gering gehalten werden, insbesondere sollte ein möglichst geringer Cholesteringehalt vorliegen. Bevorzugt werden Liposomen, die völlig frei von Cholesterin sind, da Cholesterin zu einer Verschmierung der Phasenumwandlungstemperatur führt und damit zu einem zu breiten thermischen Übergangsbereich.

Die Herstellung der erfindungsgemäßen thermolabilen Liposomen erfolgt in üblicher Weise durch Auflösen der Lipide, z.B. in Chloroform oder Chloroform/Wasser/Isopropanol, Abziehen des Lösungsmittels, zweckmäßig im Vakuum im Rotationsverdampfer, Tempern der Lipide mit wässrigen Lösungen der einzukapselnden Inhaltsstoffe bei Temperaturen, die über der Phasenumwandlungstemperatur liegen. Die Dauer dieser Temperungsbehandlung beträgt zweckmäßig 30 bis 60 Minuten, kann jedoch aber auch kürzer oder länger sein. Durch mehrfach wiederholte Einfrier-Auftau-Vorgänge, beispielsweise 2- bis 5-faches Einfrieren und wieder Auftauen, erfolgt eine Homogenisierung. Schließlich wird die erhaltene Lipidsuspension durch eine Membran definierter Porengröße bei einer Temperatur über der Phasenumwandlungstemperatur extrudiert, um die angestrebte Liposomengröße zu erreichen. Als Membran eignen sich beispielsweise Polycarbonatmembranen definierter Porengröße, wie 100 bis 200 nm. Schließlich kann gegebenenfalls nicht eingekapselter **Inhaltsstoff abgetrennt werden, beispielsweise durch** Säulenchromatographie o.dgl.

Die folgenden Abbildungen und Beispiele erläutern die Erfindung weiter.

Abbildung 1 zeigt die erhaltenen Werte der in vitro CF-Freisetzung aus thermolabilen Liposomen.

### Liposomenzusammensetzung:

DPPG:DSOC:DPPG2 = 3:2:5

Große Stabilität in Gegenwart von Serum bei 37 C (CF-Freisetzung nach 18 Stunden < 7 %).

Abbildung 2 zeigt die Beeinflussung der Freisetzungstemperatur von DDPG₂/DSPC/DPPC-Liposomen durch Variation des Anteils des DSPC auf Kosten von DPPC.

Abbildung 3 zeigt die Verbesserung der CF-Freisetzung aus DPDG₂/DSPC/DPPC-Liposomen durch Erhöhung des Anteils an DPPG₂ auf Kosten von DPPC (konstanter Anteil an DSPC mit 20 %).

Abbildung 4 zeigt die Photonenkorrelationsspektroskopie (PCS) von Liposomen aus 30 Gew.-% DPPG₂, 20 Gew.-% DSPC und 50 Gew.-% DPPC (mittlere Größe: 175 nm).

### Beispiel 1

a) In der oben beschriebenen Weise werden die in der Tabelle 2 aufgeführten Liposomen hergestellt.

**Tabelle 2**

| | | | |
|---|---|---|---|
| DPPG₂ 30 % | DSPC 0 % | DPPC 70 % | |
| DPPG₂ 30 % | DSPC 10 % | DPPC 60 % | |
| DPPG₂ 30 % | DSPC 20 % | DPPC 50 % | |
| DPPG₂ 30 % | DSPC 30 % | DPPC 40 % | |
| | | | |
| DPPG₂ 10 % | DSPC 0 % | DPPC 90 % | |
| DPPG₂ 10 % | DSPC 10 % | DPPC 80 % | |
| DPPG₂ 10 % | DSPC 20 % | DPPC 70 % | |
| DPPG₂ 10 % | DSPC 30 % | DPPC 60 % | |
| | | | |
| DPPG₂ 0 % | DSPC 20 % | DPPC 80 % | |
| DPPG₂ 10 % | DSPC 20 % | DPPC 70 % | |
| DPPG₂ 20% | DSPC 20 % | DPPC 60 % | |
| DPPG₂ 30 % | DSPC 20 % | DPPC 50 % | |
| DPPG₂ 40 % | DSPC 20 % | DPPC 40 % | |
| DPPG₂ 50 % | DSPC 20 % | DPPC 30 % | |
| DPPG₂ 80 % | DSPC 20 % | DPPC 0 % | |
| | | | |
| DSPG₂ 10 % | | DPPC 90 % | |
| DSPG₂ 20 % | | DPPC 80 % | |
| DSPG₂ 30 % | | DPPC 70 % | |
| | | | |
| DSPG₃ 10 % | | DPPC 90 % | |
| DSPG₃ 20 % | | DPPC 80 % | |
| | | | |
| DPPG₂ 30 % | DSPC 20 % | DPPC 40 % | 1PPC 10 % |
| DPPG₂ 30 % | DSPC 20 % | DPPC 30 % | 1PPC 20 % |
| | | | |
| DSPG₂ 20 % | | DPPC 70 % | 1SPC 10 % |
| DSPG₂ 20 % | | DPPC 60 % | 1SPC 20 % |
| | | | |
| DSPG₂ 20 % | | DPPC 70 % | Hexadecyl-PC 10 % |
| DSPG₂ 20 % | | DPPC 60 % | Hexadecyl-PC 20 % |
| | | | |
| DSPG₂ 20 % | | DPPC 70 % | Octadecyl-PC 10 % |
| DSPG₂ 20 % | | DPPC 60 % | Octadecyl-PC 20 % |
| | | | |
| DSPG₂ 10 % | | DPPC 80 % | Et-18 OCH₃PC 10 % |
| DSPG₂ 10 % | | DPPC 70 % | Et-18 OCH₃PC 20 % |
| DSPG₂ 10 % | | DPPC 60 % | Et-18 OCH₃PC 30 % |

| Abkürzungen: | | | |
|---|---|---|---|
| DDPC = | 1,2-Dipalmitoyl-sn-glycero-3-phosphocholin | | |
| DSPC = | 1,2-Distearoyl-sn-glycero-3-phosphocholin | | |
| DPPG₂ = | 1,2-Dipalmitoyl-sn-glycero-3-phospho-diglycerin | | |
| DSPG₂ = | 1,2-Distearoyl-sn-glycero-3-phospho-diglycerin | | |
| DSPG₃ = | 1,2-Distearoyl-sn-glycero-3-phospho-trig lycerin | | |
| 1 PPC = | 1-Palmitoyl-sn-glycero-3-phosphocholin | | |
| 1 SPC = | 1-Stearoyl-sn-glycero-3-phosphocholin | | |
| Et-18 OCH₃PC = | 1-Octadecyl-2-methyl-glycero-3-phosphocholin | | |

Sie enthalten eingekapseltes Carboxyfluorescein. Freies Carboxyfluorescein wurde vorher durch Säulenchromatographie mit Sephadex G75 abgetrennt.
b) Chamber Modell:
Zur intravitalmikroskopischen Detektion der Caroxyfluorescein (CF)-Freisetzung aus thermolabilen Liposomen im Hyperthermiefeld eignet sich das Chambermodell des syrischen Hamsters (A-Mel-3-Melanom des syrischen Hamsters). Hierbei wird einem syrischen Goldhamster eine transparente, dorsale Hautkammer implantiert. Nach Implantation der Hautkammer erfolgt die Implantation von Zellen des A-Mel-3-Melanoms des Hamsters auf das in der Kammer befindliche Subkutangewebe. Innerhalb von wenigen Tagen wächst innerhalb der Rückenhaut des Hamsters ein Tumor von mehreren Millimeter Größe. Die Mikrozirkulation sowie die Fluoreszenzanreicherung innerhalb des Tumors kann mit einem modifizierten Vitalmikroskop beobachtet werden. Die Tiere erhalten zusätzlich einen zentralen Venenkatheter. Mit Hilfe eines unter der Hautkammer befindlichen Wärmetauschers kann lokal eine Erwärmung des Tumors auf 42 °C erreicht werden. Die Tumortemperatur kann mit Hilfe einer Temperatursonde direkt gemessen werden (Endrich, 1988).

Neben der Vitalmikroskopie ist auch das Verfahren der MRT-Messung am Chamber-Modell etabliert (Pahernik et al., 1999). Hierbei können analog zur Mikroskopie MRT-Bilder aufgenommen werden.

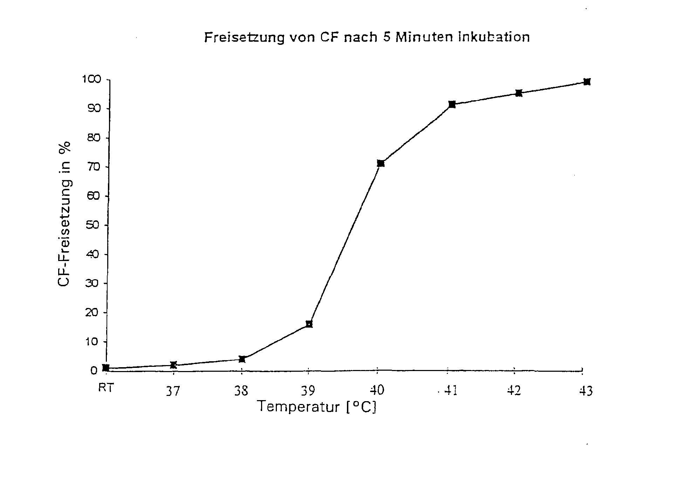

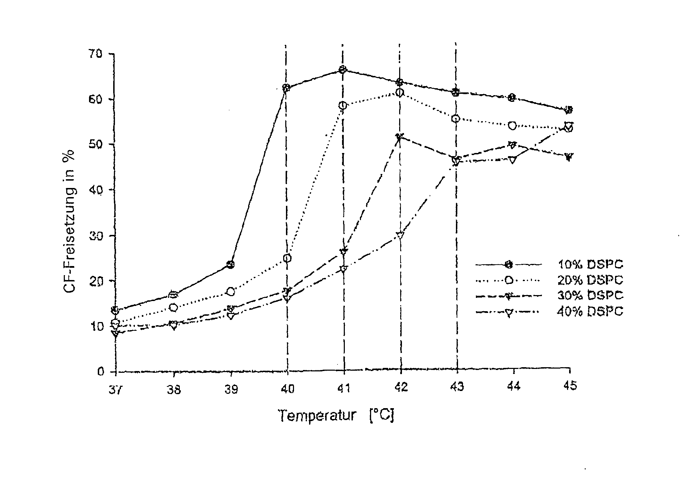

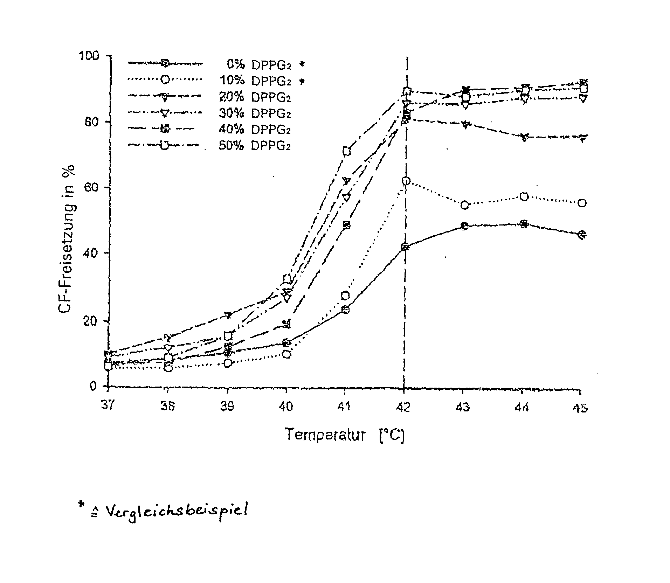

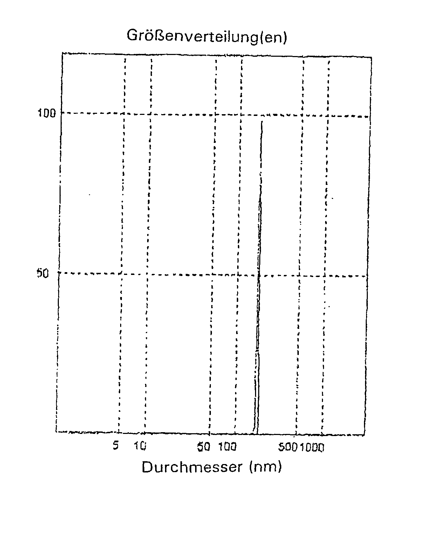

Die erhaltenen Werte der in vitro CF-Freisetzung sind in Abbildung 1 gezeigt. Ferner ist die Beeinflussung der Freisetzungstemperatur von DPPG₂/DSPC/DPPC-Liposomen durch Variation des Anteils an DSPC auf Kosten von DPPC in Abbildung 2 gezeigt. Die Verbesserung der CF-Freisetzung aus DPPG₂/DSPC/DPPC-Liposomen durch Erhöhung des Anteils an DPPG₂ auf Kosten von DPPC (konstanter Anteil an DSPC mit 20 %) ist **in** **Abbildung 3** **gezeigt. Überdies ist eine** Photonenkorrelationsspektroskopie von DPPG₂/DSPC/DPPC-Liposomen in Abbildung 4 gezeigt.

## Patentansprüche

1. Thermolabiles Liposom mit geregelter Freigabetemperatur für den Liposomeninhalt,
**dadurch gekennzeichnet,**
**dass** es im Wesentlichen aus mindestens einem Phosphatidylcholin mit einer Hauptumwandlungstemperatur im Bereich von 0 bis 80 °C und mehr als 15 bis 70 Gew.-% Phosphatidyloligoglycerin gebildet ist und dass es kein Cholesterin enthält.

2. Liposom nach Anspruch 1,
**dadurch gekennzeichnet,**
**dass** es mindestens ein Phosphatidylcholin, ausgewählt aus der Gruppe, bestehend aus 1-Palmitoyl-2-olioylglycero-3-phosphocholin, 1-Stearoyl-2-olioyl-3-phosphocholin, 1-Palmitoyl-2-lauroylglycero-3-phosphocholin, 1-Behenoyl-2-olioylglycero-3-phosphocholin, 1-Stearoyl-2-lauroylglycero-3-phosphocholin, 1,3-Dimyristoylglycero-2-phosphocholin, 1,2-Dimyristoylglycero-3-phosphocholin, 1-Palmitoyl-2-myristoylglycero-3-phosphocholin, 1-Stearoyl-2-myristoylglycero-3-phosphocholin, 1-Myristoyl-2-palmitoylglycero-3-phosphocholin, 1,3-Palmitoylglycero-2-phosphocholin, 1,2-Dipalmitoylglycero-3-phosphocholin, 1-Myristoyl-2-stearoylglycero-3-phosphocholin, 1-Stearoyl-3-myristoylglycero-2-phosphocholin, 1-Stearoyl-2-palmitoylglycero-3-phosphocholin, 1-Palmitoyl-2-stearoylglycero-3-phosphocholin, 1,3-Distearoylglycero-2-phoshocholin, 1,2-Distearoylglycero-3-phosphocholin, 1,2-Diarachinoylglycero-3-phosphocholin, 1,2-Dibehenoylglycero-3-phosphocholin und 1,2-Dilignoceroylglycero-3-phosphocholin, enthält.

3. Liposom nach Anspruch 1 oder 2,
**dadurch gekennzeichnet,**
**dass** es als Phosphatidyloligoglycerin Dipalmitoylphosphoglyceroglycerin enthält.

4. Liposom nach einem der vorhergehenden Ansprüche,
**dadurch gekennzeichnet,**
**dass** es im Wesentlichen aus 20 bis 75 % Dipalmitoyllecithin (DPPC), 10 bis 25 % Distearoyllecithin (DSPC) und mehr als 15 bis 50 % Dipalmitoylphosphoglyceroglycerin (DPPG2) besteht.

5. Liposom nach einem der vorhergehenden Ansprüche,
**dadurch gekennzeichnet,**
**dass** es zusätzlich bis 15 % mindestens eines Alkylphosphocholins enthält.

6. Liposom nach Anspruch 5,
**dadurch gekennzeichnet,**
**dass** es 10 bis 15 % mindestens einer der Verbindungen Hexadecylphosphocholin, Oleoylphosphocholin oder Etherlysolecithin enthält.

7. Liposom nach einem der vorhergehenden Ansprüche,
**dadurch gekennzeichnet,**
**dass** es einen Wirkstoff oder/und eine Markierungssubstanz enthält.

## Claims

1. Thermolabile liposome having a controlled release temperature for the liposome content, **characterised in that** it is formed of at least one phosphatidylcholine having a main transition temperature in the range of from 0 to 80 °C and more than 15 to 70 wt.% phosphatidyl-oligo-glycerol, and **in that** it does not contain any cholesterol.

2. Liposome according to claim 1, **characterised in that** it contains at least one phosphatidylcholine selected from the group consisting of 1-palmitoyl-2-olioylglycero-3-phosphocholine, 1-stearoyl-2-olioyl-3-phosphocholine, 1-palmitoyl-2-lauroyl-glycero-3-phosphocholine, 1-behenoyl-2-olioyl-glycero-3-phosphocholine, 1-stearoyl-2-lauroyl-glycero-3-phosphocholine, 1,3-dimyristoylglycero-2-phosphocholine, 1,2-dimyristoylglycero-3-phosphocholine, 1-palmitoyl-2-myristoylglycero-3-phosphocholine, 1-stearoyl-2-myristoylglycero-3-phosphocholine, 1-myristoyl-2-palmitoylglycero-3-phosphocholine, 1,3-palmitoylglycero-2-phosphocholine, 1,2-dipalmitoylglycero-3-phosphocholine, 1-myristoyl-2-stearoylglycero-3-phosphocholine, 1-stearoyl-3-myristoylglycero-2-phosphocholine, 1-stearoyl-2-palmitoylglycero-3-phosphocholine, 1-palmitoyl-2-stearoylglycero-3-phosphocholine, 1,3-di-stearoylglycero-2-phosphocholine, 1,2-di-stearoylglycero-3-phosphocholine, 1,2-di-arachinoylglycero-3-phosphocholine, 1,2-di-behenoylglycero-3-phosphocholine and 1,2-di-lignoceroylglycero-3-phosphocholine.

3. Liposome according to either claim 1 or claim 2, **characterised in that** said phosphatidyl-oligo-glycerol is dipalmitoylphosphoglyceroglycerol.

4. Liposome according to any of the preceding claims, **characterised in that** it substantially consists of from 20 to 75 % dipalmitoyl lecithin (DPPC), 10 to 25 % distearoyl lecithin (DSPC) and more than 15 to 50 % dipalmitoylphosphoglyceroglycerol (DPPG2).

5. Liposome according to any of the preceding claims, **characterised in that** it additionally contains up to 15 % of at least one alkylphosphocholine.

6. Liposome according to claim 5, **characterised in that** it contains from 10 to 15 % of at least one of the compounds hexadecylphosphocholine, oleoyl-phosphocholine or ether lysolecithin.

7. Liposome according to any of the preceding claims, **characterised in that** it contains an active substance and/or a tracer.

## Revendications

1. Liposome thermolabile à température de libération régulée pour le contenu du liposome,
**caractérisé en ce**
**qu'**il est formé essentiellement d'au moins une phosphatidylcholine présentant une température de conversion principale dans la plage de 0 à 80 °C et plus de 15 à 70 % en poids de phosphatidyloligoglycérine et qu'il ne contient pas de cholestérol.

2. Liposome selon la revendication 1,
**caractérisé en ce**
**qu'**il contient au moins une phosphatidylcholine choisie dans le groupe constitué de la 1-palmitoyl-2-olioylglycéro-3-phosphocholine, la 1-stéaroyl-2-olioyl-3-phosphocholine, la 1-palmitoyl-2-lauroylglycéro-3-phosphocholine, la 1-béhénoyl-2-olioylglycéro-3-phosphocholine, la 1-stéaroyl-2-lauroylglycéro-3-phosphocholine, la 1,3-dimyristoylglycéro-2-phosphocholine, la 1,2-dimyristoylglycéro-3-phosphocholine, la 1-palmitoyl-2-myristoylglycéro-3-phosphocholine, la 1-stéaroyl-2-myristoylglycéro-3-phosphocholine, la 1-myristoyl-2-palmitoylglycéro-3-phosphocholine, la 1,3-palmitoyl-glycéro-2-phosphocholine, la 1,2-dipalmitoylglycéro-3-phosphocholine, la 1-myristoyl-2-stéaroylglycéro-3-phosphocholine, la 1-stéaroyl-3-myristoylglycéro-2-phosphocholine, la 1-stéaroyl-2-palmitoylglycéro-3-phosphocholine, la 1-palmitoyl-2-stéaroylglycéro-3-phosphocholine, la 1,3-distéaroylglycéro-2-phosphocholine, la 1,2-distéaroylglycéro-3-phosphocholine, la 1,2-diaraquinoylglycéro-3-phosphocholine, la 1,2-dibéhénoylglycéro-3-phosphocholine et la 1,2-dilignocéroylglycéro-3-phosphocholine.

3. Liposome selon la revendication 1 ou 2,
**caractérisé en ce**
**qu'**il contient, comme phosphatidyloligoglycérine, de la dipalmitoylphosphoglycéroglycérine.

4. Liposome selon l'une des revendications précédentes,
**caractérisé en ce**
**qu'**il est constitué essentiellement de 20 à 75 % de dipalmitoyllécithine (DPPC), de 10 à 25 % de distéaroyllécithine (DSPC) et de plus de 15 à 50 % de dipalmitoylphosphoglycéroglycérine (DPPG2).

5. Liposome selon l'une des revendications précédentes,
**caractérisé en ce**
**qu'**il contient en outre jusqu'à 15 % d'au moins une alkylphosphocholine.

6. Liposome selon la revendication 5,
**caractérisé en ce**
**qu'**il contient 10 à 15 % d'au moins l'un des composés hexadécylphosphocholine, oléoylphosphocholine ou étherlysolécithine.

7. Liposome selon l'une des revendications précédentes,
**caractérisé en ce**
**qu'**il contient une substance active et/ou une substance de marquage.
